# EUROPEAN PATENT APPLICATION

(11) **EP 4 345 085 A1**
(43) Date of publication of application: **03.04.2024**
(21) Application number: 23199314.8
(22) Date of filing: 25.09.2023
(51) Int. Cl.: C07C 1/04, C07C 15/04, C01B 3/34, C07C 201/08, C07C 205/06, C07C 209/36, C07C 211/48, C07D 277/72

(54) **RUBBER ADDITIVE FROM BIOMASS THROUGH SYNGAS PRODUCTION**

(30) Priority: 28.09.2022 US 202217936189
(71) Applicant: The Goodyear Tire & Rubber Company, Akron, OH 44316 (US)
(72) Inventor: YANG, Xiaoping, Streetsboro, 44241 (US); HUA, Kuo-Chih, Richfield, 44286 (US); BENKO, David Andrew, Munroe Falls, 44262 (US)
(74) Representative: Kutsch, Bernd

(57) **Abstract**

A method of synthesizing an additive for a tire is disclosed. The method comprises: converting biomass to syngas or to a gaseous mixture predominately comprising carbon monoxide and hydrogen; synthesizing at least one of benzene and an alkyl-substituted benzene from carbon monoxide and hydrogen in said syngas or said gaseous mixture; synthesizing at least one of aniline and an alkyl-substituted aniline from the at least one of the benzene and the alkyl-substituted benzene; and synthesizing the additive from the at least one of the aniline and the alkyl-substituted aniline. Also, a method of forming a curing system, a method of forming a tire or a tire component and a system for synthesizing an additive for a tire is disclosed.

## Description

### Background

The invention relates to a method and system for conversion of biomass to starting materials useful in the formation of tires.

Pneumatic tires are commonly produced from rubber monomers, fillers, reinforcing materials, plasticizers, vulcanization chemicals, antiaging chemicals, and the like. The rubber monomers, as well as other tire components, are generally derived from petroleum. Since tires have a short lifetime of a few years, this leads to consumption of considerable amounts of non-renewable resources. It would therefore be desirable to reduce the reliance on fossil fuels in the formation of tires.

Biomass includes a wide range of plant-based materials, particularly cellulosic and starch-based materials, including energy crops, such as switch grass and miscanthus, agricultural sources such as corn husks, wood pellets, lumbering and timbering wastes, yard wastes, construction, and demolition waste, and biosolids (treated sewage sludge). Unlike fossil fuels, biomass provides a renewable resource of energy, since it is rapidly regenerated within months or years, rather than millennia. Compared with fossil fuels, however, biomass contains a substantial amount of water.

One route for obtaining useful products from biomass is through fermentation, which yields ethanol. In turn, the ethanol can be converted to tire-forming materials, such as butadiene. However, for lignocellulosic plant biomass, chemical or enzymatic pretreatment is generally needed to solubilize the biomass prior to microbial bioconversion. This increases the cost of the overall conversion process.

There remains a need for a system and method for converting biomass into feedstock materials for producing tires in an efficient manner that does not impact the quality of the tires produced. The exemplary system and method provide a value-added route for converting waste materials into high quality feedstocks for new tires which can reduce reliance on non-renewable resources.

US 9,115,047 B2 describes a method for synthesis of rubber chemicals through a process which includes a catalytic conversion of ethanol to an aromatic compound (benzene, toluene, xylene, ethylbenzene, diethylbenzene, or butylbenzene) and a further conversion of the aromatic compound to aniline and/or styrene.

US 9,663,445 B2 describes a method for synthesizing aniline from a biomass material via phenol.

US 9,598,552 B2 describes rubber compositions containing carbon black produced from a feedstock oil.

### Summary of the Invention

The invention relates to a method in accordance with claim 1, 13 or 14 and to a system in accordance with claim 15.

Dependent claims refer to preferred embodiments of the invention.

In accordance with a preferred embodiment, a method of forming a tire-forming additive includes converting biomass to syngas. Benzene and/or an alkyl-substituted benzene is synthesized from carbon monoxide and hydrogen in the syngas. Aniline and/or an alkyl-substituted aniline is synthesized from the benzene and/or alkyl-substituted benzene. A tire-forming additive is synthesized from the aniline and/or alkyl-substituted aniline. The tire-forming additive is selected from the group consisting of an anti-degradant, a vulcanization accelerator, and combinations thereof.

In accordance with another preferred embodiment, a system for forming a tire forming additive includes a first reactor in which biomass is converted to syngas; a second reactor fluidly connected to the first reactor and configured for synthesizing at least one of benzene and an alkyl-substituted benzene from carbon monoxide and hydrogen in the syngas; a third reactor fluidly connected to the second reactor and configured for synthesizing at least one of aniline and an alkyl-substituted aniline from the at least one of the benzene and the alkyl-substituted benzene; and a fourth reactor fluidly connected to the third reactor and configured for synthesizing a tire-forming additive from the at least one of the aniline and the alkyl-substituted aniline. The tire-forming additive is selected from the group consisting of an anti-degradant, a vulcanization accelerator, and combinations thereof.

In accordance with another preferred embodiment, a method of forming a tire or component thereof includes converting biomass to syngas; synthesizing at least one of benzene and an alkyl-substituted benzene, from carbon monoxide and hydrogen in the syngas; synthesizing at least one of aniline and an alkyl-substituted aniline from the at least one of the benzene and the alkyl-substituted benzene; synthesizing at least one tire-forming additive from the at least one of the aniline and the alkyl-substituted aniline, the at least one tire-forming additive being selected from the group consisting of an anti-degradant, a vulcanization accelerator, and combinations thereof; and combining the at least one tire-forming additive with a rubber polymer, which may be formed from carbon monoxide and hydrogen in the syngas.

### Brief Description of the Drawings

FIGURE 1 is a schematic diagram of a pathway for forming tires from biomass;
FIGURE 2 illustrates a method for conversion of biomass to feedstock materials for forming tires; and
FIGURE 3 illustrates a system for conversion of biomass to tire-forming additives.

### Detailed Description of Preferred Embodiments of the Invention

A system and method are described for generating feedstocks for tire manufacture from biomass. The biomass employed in the method may include homogeneous biomass materials (biomass materials which come from a single source) or heterogeneous biomass materials (biomass materials which come from multiple sources). Examples of homogeneous biomass materials include coniferous trees or deciduous trees of a single species, agricultural materials from a plant of a single species, such as hay, corn husks, wheat, switch grass, miscanthus, primary sludge from wood pulp, wood chips, seed oils, plant fruits, waste cooking oil, lychee fruit bark, and the like. Heterogeneous biomass materials include forest residues from mixed species, tree residues from mixed species obtained from debarking operations or sawmill operations, compost, yard wastes, construction waste, demolition waste, and biosolids (treated sewage sludge).

As will appreciated, depending on the source of the biomass, some inorganic materials and other contaminants may be present in the biomass, such as metals, contaminants such as sulfur, halogens, or non-biomass nitrogen contained in compounds such as inorganic salts or organic compounds.

The illustrated approach enables conversion of biomass into high quality feedstocks that are substantially the same as the feedstock materials obtained from petroleum sources. As a result, the rubber chemicals produced have the same chemical composition as the petroleum sourced counterparts, so that no property or performance deficit is expected. This approach reduces waste and provides a more efficient use of natural resources.

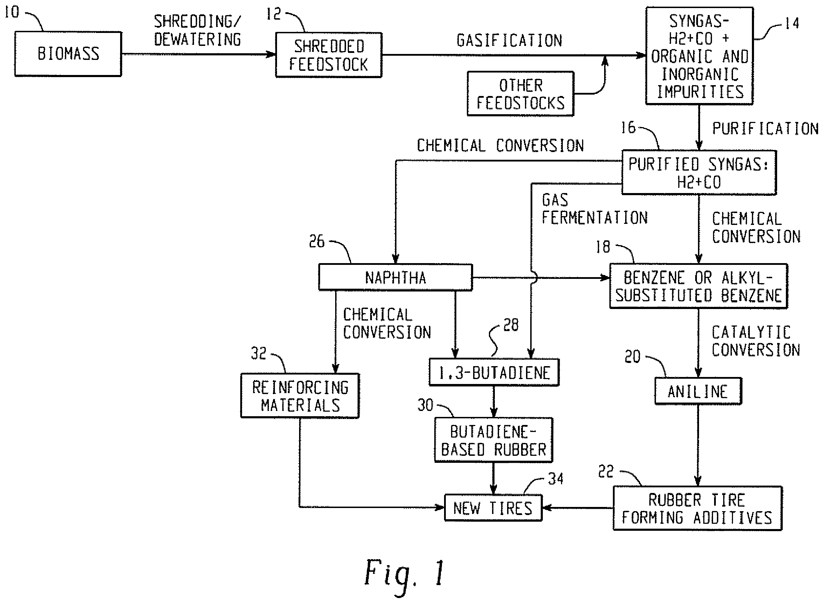

With reference to FIGURE 1, an overview of a pathway for biomass conversion is illustrated. The bulk starting material is raw biomass 10, e.g., one of the sources described above. The starting material 10 may thus include a substantial amount of water, such as from 1-99 wt. % water, e.g., at least 2 wt. %, or at least 5 wt. %.

The starting material 10 is preprocessed to form a biomass feedstock 12. This may include pulverizing, shredding, chipping, grinding or otherwise comminuting the solid material, reducing the water content, e.g., to below 5 wt. %, and/or removing larger impurities, such as pieces of metal. The feedstock 12 is converted to a reaction product 14 through a gasification process. Additionally, other feedstocks may be subjected to the gasification process. The reaction product 14 is predominantly a gaseous mixture of carbon monoxide and hydrogen, known as synthetic gas or "syngas". The reaction product 14 further includes organic and inorganic impurities, i.e., components other than hydrogen gas and carbon monoxide gas, in amounts depending on the bulk starting material 10. These impurities may include significant amounts of carbon dioxide (CO₂) and water (H₂O). The impurities are removed, or at least reduced, to generate a purified syngas 16. The purified syngas includes hydrogen gas and carbon monoxide gas and optionally minor amounts of impurities, e.g., from 0-5 wt. % impurities or no more than 1 wt.% impurities.

At least a portion of the purified syngas 16 is converted to benzene 18 (and/or to an alkyl-substituted benzene) through a chemical conversion process. The benzene 18 (and/or the alkyl-substituted benzene) is converted to aniline 20 (and/or an alkyl-substituted aniline). The aniline 20 (and/or the alkyl-substituted aniline) is converted to one or more rubber forming additives 22, such as anti-degradants (e.g., antioxidants and antiozonants) and vulcanization accelerators.

Optionally, at least a portion of the purified syngas 16 is converted to one or more other rubber-forming monomers, such as one or more hydrocarbons, such as a C₁-C₁₀ alkene, e.g., butadiene, isoprene, and/or isobutylene; a vinylarene, such as styrene; and/or a mixture of liquid hydrocarbons, such as naphtha. Other rubber-forming monomers include C₁-C₁₀ alcohols. Mixtures of such rubber-forming monomers may be formed.

In one embodiment, the rubber-forming monomer includes naphtha 26, e.g., formed through a Fischer-Tropsch process. Optionally, at least a portion of the naphtha 26 may be converted to 1,3-butadiene 28, which can be used as a monomer for forming a synthetic rubber polymer, in particular, a butadiene-based rubber 30. Optionally, at least a portion of the naphtha may be converted to reinforcing materials 32, such as polyethylene terephthalate (PET)-based or nylon-based fabrics and cords. Additionally, or alternatively, the naphtha may be converted to benzene.

The syngas-derived products 22, 30, 32 can be used as feedstocks to form tires 34 or components thereof.

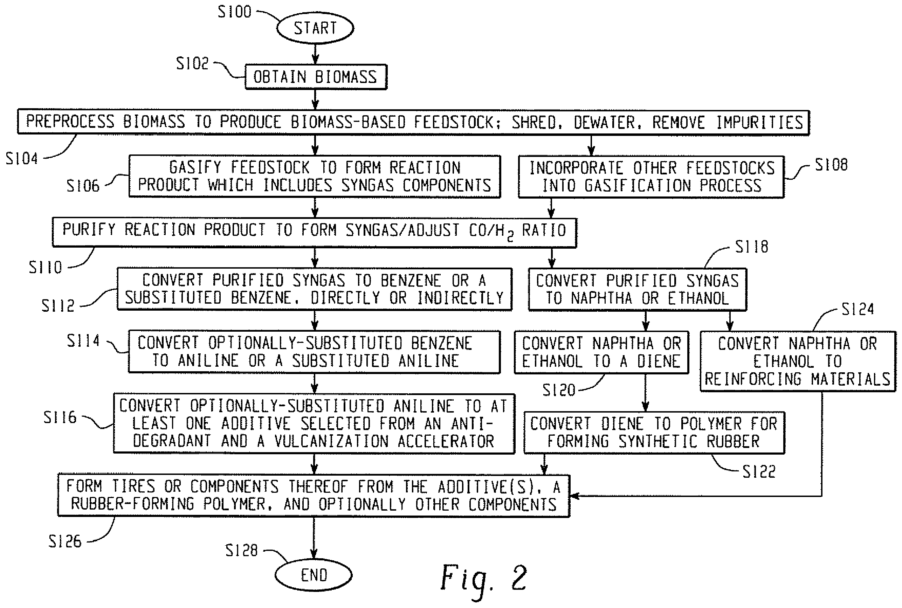

With reference to FIGURE 2, a method for forming feedstock materials for tire production is illustrated. The method begins at S100. At S102, biomass is obtained as a starting material 10.

At S104, the biomass starting material 10 may be preprocessed, which may include comminuting the solids, reducing the water content, reducing impurities, or the like to produce a preprocessed biomass feedstock 12.

At S106, the biomass feedstock 12 is subjected to a gasification process (or other suitable reaction process) which converts the comminuted biomass starting material 10 to a reaction product 14 containing carbon monoxide gas and hydrogen gas.

Optionally, at S108, other feedstocks may be subjected to the gasification process.

At S110, the reaction product 14 may be purified to remove organic and inorganic impurities, i.e., components other than hydrogen gas and carbon monoxide gas, resulting in a (relatively) pure syngas 16, e.g., one in which carbon monoxide and hydrogen constitute at least 70 wt. % or at least 80 wt.% or at least 90 wt. % of the gas and/or in which other inorganic materials constitute no more than 5 wt. %, or no more than 1 wt. %, or no more than 0.2 wt. %.

At S112, at least some of the purified syngas 16 may be converted, directly or indirectly, to benzene 18 and/or an alkyl-substituted benzene through a chemical conversion process.

At S114, the optionally-substituted benzene 18 is converted to aniline 20 and/or an alkyl-substituted aniline.

At S116, the optionally-substituted aniline 20 is converted to one or more rubber-forming additives 22, such as antioxidants, antiozonants, and vulcanization accelerators (curing agents).

At S118, optionally, at least some of the purified syngas 16 may be converted to naphtha 26, e.g., through a Fischer Tropsch process, or to ethanol, e.g., through a gas fermentation process.

At S120, at least a portion of the naphtha or ethanol 26 produced at S118 may be converted to a diene compound, e.g., 1,3-butadiene 28.

At S122, at least a portion of the 1,3-butadiene 28, or other diene produced at S120, may be converted to a homopolymer or copolymer, such as a butadiene-based rubber-forming polymer 30.

At S124, at least a portion of the naphtha or ethanol 26 produced at S118 may be converted to reinforcing materials 32, such as polyethylene terephthalate-based materials or nylon-based materials, e.g., fabric and cords for use as tire components.

At S126, one or more of the syngas-derived products 30, 32, 22 may be used as a feedstock to form new tires 34 or components thereof, such as tire sidewalls and/or tire treads.

The method ends at S128, or may return to S102, where the tires, once worn, are subsequently reclaimed and used as another feedstock at S108. Further details of the method will now be described.

### Preprocessing of Biomass (S104)

In this step, the biomass is optionally preprocessed, to reduce the time and/or energy needed for gasification, and/or to improve the quality of the reaction product.

### Syngas Generation (S106)

Gasification can be performed with steam (e.g., derived from the biomass), carbon monoxide, oxygen (e.g., as air), or a mixture thereof. The reaction temperature may be at least 700°C, or up to 1000°C, or higher. The pressure may be atmospheric or above. High pressure gasification may be conducted at 0.1MPa or higher, such as up to 0.5 MPa.

The gasification process proceeds through a number of stages, primarily drying, pyrolysis, oxidation, and reduction. Drying may be performed at 100°C - 200°C. This is followed by pyrolysis, where the thermal decomposition of biomass occurs in the absence of oxygen, releasing hydrocarbon gases. The biomass is reduced to carbonized biomass. Subsequently, CO, H₂, CH₄, and CO₂ gases as well as water and tars are formed. Carbon monoxide can be predominantly generated (relative to carbon dioxide) provided oxygen is present in sub-stoichiometric quantities, leading to the carbon being only partially oxidized.

Most of the endothermic reduction reactions occur at 800°C to 1000°C, in the absence of oxygen. The feedstock composition, reactor type, and operating parameters (temperature, pressure and oxygen-fuel ratio) are able to determine the composition of the gas and the level of undesirable components (tars, dust, ash content) produced during gasification process,

Several types of gasifier configurations are available for gasification of waste starting materials: These include: (1) A fixed-bed gasifier, which can be a countercurrent fixed-bed gasifier or a co-current fixed-bed gasifier; (2) A fluidized bed gasifier; (3) An entrained-flow gasifier; and (4) A plasma gasifier.

Each type of gasifier can be designed to operate either at atmospheric pressure or at higher pressure. In the high-pressure type of operation, the hydrogasification (gasification in a hydrogen-rich environment) process is optimized and the quality of the product gas can be improved. In order to accommodate different feedstocks and different process requirements, there are several gasifier designs which are distinguished by: (i) the use of wet or dry feedstock, (ii) the use of air or oxygen, (iii) the flow direction within the gasifier - up-flow, down-flow, or circulating flow, and (iv) the cooling process for the syngas and other gaseous products. Further details on gasification methods are to be found in James G. Speight, "Synthesis Gas: Production and Properties," John Wiley & Sons, Inc. (2020).

In fluidized bed gasifiers, gasification temperatures between 800 and 900°C for a period of three to ten hours may be employed. The biomass may be gasified with superheated steam (e.g., heated with waste heat from the reaction). The biomass may first be converted to char at high temperatures, followed by reaction of the char with the superheated steam.

Plasma gasification is also suited to the gasification of biomass. Plasma is used in two different ways in the gasification process, first as a heat source during gasification and second for tar cracking after standard gasification. While the primary feedstock discussed herein is biomass, the feedstock can be combined with other feedstocks, such as rubber materials and plastics. Compared to other traditional gasification processes, plasma gasification allows the feedstocks to be gasified at very high temperatures, which eliminates/reduces tar formation and can achieve high conversion. The high operating temperatures break down the feedstock and/or all hazardous and toxic components into their respective elemental constituents, and dramatically increases the kinetics of the various reactions occurring in the gasification zone, converting all organic materials into hydrogen and carbon monoxide. Because the feedstocks reacting within the gasifier are converted into their basic elements, even hazardous waste becomes a useful syngas. Inorganic materials in the feedstock are melted and fused into a glassy-like slag, which is nonhazardous and can be used in a variety of applications, such as roadbed construction and roofing materials.

Plasma gasification provides several benefits, including greater feedstock flexibility (for example, feedstocks can be mixed, such as municipal solid waste, other biomass, tires, hazardous waste, and auto shredder waste); a high conversion (>99%) of carbonaceous matter to syngas; absence of tar in the synthesis; production of little or no char, ash, or residual carbon; and low carbon dioxide emissions.

As a result, at least 70 wt.%, or at least 80 wt.%, or 95 wt.% of the feedstock can be transformed into syngas, with the remainder being an inert ash (inorganics), which can be removed.

### Incorporation of Other Feedstocks (S108)

The method is not limited to the gasification of biomass. Other feedstocks that are rich in the elements hydrogen and carbon may also be gasified, separately or together with the biomass. Such feedstocks may include rubber products, such as tires, rubber-based hoses, plastics, and the like. In the exemplary embodiment, a ratio, by dry weight, of these other feedstocks, in total, to the biomass feedstock used in syngas production is less than 1:1.

### Purification of the Reaction Product (S110)

The syngas 14 as produced by the various syngas production processes may be saturated with water vapor and contain a mixture of hydrogen, carbon monoxide, carbon dioxide, methane as well as other contaminants, such as hydrogen sulfide (H₂S), and other trace components. Accordingly, the reaction product 14 is generally dried, scrubbed to remove gaseous impurities and filtered to remove solid reaction products.

In addition, the H₂/CO molar ratio in the syngas may be adjusted to meet the requirements of the downstream processes. These downstream processes may include forming one or more of a wide range of gaseous, liquid, and/or solid feedstocks, including benzene.

Since different downstream processes benefit from specific syngas compositions (as well as limits to other contaminants), the syngas may be conditioned by one or more of the following processes:

### Water Gas Shift:

To increase the H₂/CO molar ratio, a water gas shift reaction may be used to convert a portion of the CO content in the syngas to CO₂ and additional H₂:

*CO* + *H₂O* → *CO*₂ + *H₂* (1)

A dedicated high pressure water gas shift reactor and a suitable catalyst may be employed. See, for example, Erlisa Baraj, et al., "The water gas shift reaction: Catalysts and reaction mechanism," Fuel, Vol. 288, 119817 (15 March 2021).

### Acid Gas Removal (AGR):

This is used for sulfur contaminant and/or CO₂ removal and may be performed with cyclic, regenerable, and/or solvent absorption processes. In these processes, a liquid solvent is counter-currently contacted with the syngas in an absorption tower to selectively remove the H₂S and/or the CO₂. It is then thermally regenerated in a stripping tower to liberate the acid gases while rejuvenating the solvent to begin the absorption cycle again.

A dedicated AGR unit may be provided for this purpose.

### Methanation:

This reaction converts carbon oxides and hydrogen to methane and water:

*CO* + *3H₂* → *CH₄* + *H₂O* (2)

*CO₂* + *4H₂* → *CH₄* + *2H₂O* (3)

The methanation reactions may be catalyzed by a nickel catalyst. In the methanol industry, there are two typical uses for methanation: to purify synthesis gas and to produce methane for synthetic natural gas (SNG).

A dedicated methanation unit may be provided for this purpose.

### Pressure Swing Adsorption (PSA):

This process may be used to separate and purify the syngas stream. In one embodiment, a pure hydrogen stream is generated, which can then be used to modify the H₂/CO molar ratio and/or serve as a reactant for forming materials for forming tires. The hydrogen stream resulting from the PSA process can be at least 99 wt. % hydrogen, or at least 99.9 wt. % hydrogen, for example, purities of 99.999% hydrogen can be achieved.

A PSA unit may be employed which utilizes a timed cycle of steps of adsorption, pressure equalization, depressurization, blowdown, purge, and re-pressurization across multiple beds loaded with various types of adsorbent (e.g., activated alumina, silica gel, activated carbon, molecular sieves).See, for example, Dong-Kyu Moon, et al., "H2 pressure swing adsorption for high pressure syngas from an integrated gasification combined cycle with a carbon capture process, Applied Energy, Vol. 183, pp. 760-774 (December 2016).

### Membrane Adjustment:

A membrane unit can be used in a method to adjust the H₂/CO molar ratio in the syngas stream for use in downstream processes. In one embodiment, the adjustment is used to reduce the H₂/CO molar ratio. In another embodiment, the adjustment is used to increase the H₂/CO molar ratio, e.g., to produce a relatively high purity stream of H₂ or CO.

In an example membrane unit, the syngas is cooled to remove any condensable hydrocarbons or water, and then heated before entering the membrane. In the membrane, hydrogen permeates preferentially through the membrane, producing a purified hydrogen stream. This results in the remaining stream having a lower, or adjusted, H₂/CO molar ratio.

### Cryogenic Separation:

This can be used for the separation of CO from a syngas stream and/or to adjust (e.g., decrease) the H₂/CO molar ratio. The syngas is first pretreated to remove any impurities that could freeze at cryogenic temperatures, primarily water and carbon dioxide. The components of the syngas are then separated using differences in their boiling points.

### Removal of Soot and Other Solid Byproducts:

In this process, particulate matter (PM) in the syngas stream (such as ash, unreacted carbon in the form of tar (sticky residues) or char (hard solid deposits), and condensed chlorine and alkali compounds) is removed/reduced. PM can be removed using one or more wet or dry PM removal systems, such as water quench systems, cyclones and rotating particle separators, mesh-type filters; candle filters (ceramic or sintered metal), water and/or oil scrubbers, electrostatic precipitators (wet or dry), and combinations thereof.

For example, syngas from pyrolysis reactors and gasifiers that operate at relatively low temperatures tend to contain tar. Tar is generally a mixture of organic compounds with a high aromatic content, which can form hard-to remove deposits in downstream reactors. Tar removal processes include thermal processes, such as thermal cracking, catalytic tar cracking, and partial oxidation; and physical tar removal processes, e.g., using scrubbers, wet electrostatic methods, or precipitators.

### Removal of Mercury:

This may be performed to comply with environmental regulations and/or to avoid contamination of catalysts used in downstream processes. Mercury removal from syngas may be performed by contacting the impure syngas with sulfur-impregnated activated carbon. In one embodiment, mercury removal is performed after PM and tar removal, and prior to sulfur and CO₂ removal.

Further details on syngas treatment are provided in Global Syngas Technologies Council, "Syngas Conditioning & Purification," 2022, available at https://globalsyngas.org/syngas-technology/syngas-conditioning-purification.

### Conversion of Syngas to Materials for Forming Tires (S112-S124)

Syngas is a useful intermediate in the formation of several tire-forming materials.

As exemplified in FIGURES 1 and 2, syngas conversion to various rubber monomers and chemical additives can be performed through a gas fermentation route, a chemical reaction route, or a combination of both. The monomers that can be generated from syngas include butadiene, isoprene, styrene, isobutylene, ethylene glycol, terephthalic acid, hexamethylenediamine, and adipic acid. These monomers can be used to form synthetic rubbers (e.g., polybutadiene, styrene butadiene rubber (SBR), polyisoprene, butyl rubber, and ethylene propylene diene monomer rubber (EPDM)) and reinforcement materials (e.g., PET and Nylon-6,6).

Additionally, the syngas can be used for synthesis of rubber-forming additives, such as anti-degradants (e.g., antioxidants, antiozonants), and vulcanization accelerators, through a common intermediate, aniline.

### Synthesis of Benzene from Syngas (S112)

Aromatic hydrocarbons, such as benzene and C₁-C₆ mono, di, or tri-alkyl-substituted benzene, such as toluene, xylene, ethylbenzene, diethylbenzene, and/or butylbenzene, can be produced from syngas directly, using zeolite-based catalyst, or indirectly, via an intermediate.

Zeolite-based catalysts which may be used include ZSM catalysts (synthetic zeolites with a high silica to alumina ratio). These include the protonated form (HZSM), and those with a metal or metals as the cation (e.g., Na-ZSM, MnCr-ZSM, and the like). A common form of ZSM in these zeolites is ZSM-5 (the 5 denoting a pore diameter of 5 Å (angstroms)). ZSM-11 may also be employed.

One route for synthesizing aromatics from syngas includes a combination of catalysts, of which one may be a zeolite-based catalyst and the other may be a zinc-based catalyst. See, e.g., Zhao, et al., "Direct Transformation of Syngas to Aromatics Over Na-Zn-Fe5C2 and Hierarchical HZSM-5 Tandem Catalysts," Chem. 3(2), 323-333 (2017). The two catalysts are used in tandem, with Na-Zn-Fe₅C₂ being used to generate olefins (in particular, alkenes) and HZSM-5 (a synthetic zeolite with a high silica (Si) to alumina (Al) ratio) providing a direct conversion of syngas to aromatics. The olefins in the mixture can be separated out and used for other purposes.

Another mixed catalyst for direct conversion of syngas to aromatics is a bifunctional catalyst composed of Zn-doped ZrO₂ nanoparticles and H-ZSM-5 as described in Cheng, et al., "Bifunctional catalysts for one-step conversion of syngas into aromatics with excellent selectivity and stability," Chem 3.2: 334-347 (2017). See, also Miao, et al., "Selective Synthesis of Benzene, Toluene, and Xylenes from Syngas," ACS Catal. 10, 7389-7397 (2020), which describes use of a similar multifunction mixture of catalysts.

Benzene can also be synthesized from naphtha that is produced from syngas through a Fischer-Tropsch (FT) process. The FT process employs syngas with a H₂/CO molar ratio of close to 2:1 and a suitable catalyst. In the FT chemical reaction, carbon monoxide and hydrogen are converted into hydrocarbon derivatives of various molecular weights according to the following equation:

*(2n+1) H₂* + *nCO* → *CₙH*_{*(*2*n*+2*)*} + *nH₂O* (4),

where n is an integer.

Depending on the catalyst, temperature, and type of process employed, hydrocarbon derivatives ranging from methane to higher molecular weight paraffin derivatives and olefin derivatives can be obtained. The reaction products are fractionated into the final products of diesel, naphtha, and other light liquids, depending on the desired product or mix of products.

Naphtha refers to straight-chain, or aliphatic, hydrocarbons containing 5 to 10 carbon atoms. To reform naphtha into benzene, the naphtha is mixed with hydrogen gas at a temperature of 450 to 550°C, and the mixture is passed over a catalyst, such as platinum or rhenium, at above atmospheric pressure, e.g., in the range of 400 to 600kPa. This process converts aliphatic hydrocarbons to their corresponding aromatic compounds. Benzene can be formed from the hexane in the naphtha and separated from the other hydrocarbons in the mixture by dissolution and/or distillation.

In another embodiment, aromatics such as benzene, toluene and xylene, can be produced from syngas via either methanol or ethanol. Conversion of syngas to methanol can be performed in the gaseous or liquid phase as described, for example, in National Energy Technology Laboratory, 10.3. "Syngas Conversion to Methanol," available at https://www.netl.doe.gov/research/coal/energysystems/gasification/gasifipedia/methano l#:~:text=Methanol%20production%20from%20syngas%20is,day%20(t%2Fd).

Ethanol may be produced from syngas by fermentation. A variety of microorganisms, e.g., anaerobic bacteria, can be used for the fermentation. Examples include acetogenic bacteria, such as Acetogenium kivui, Acetoanaerobium noterae, Acetobacterium woodii, Alkalibaculum bacchi CP11, Blautia producta, Butyribacterium methylotrophicum, Caldanaerobacter subterraneous, Caldanaerobacter subterraneous pacificus, Carboxydothermus hydrogenoformans, Clostridium aceticum, Clostridium acetobutylicum, Clostridium acetobutylicum P262, Clostridium autoethanogenum, Clostridium carboxidivorans P7, Clostridium coskatii, Clostridium drakei, Clostridium Ijungdahlii PETC, Clostridium Ijungdahlii ER12, Clostridium Ijungdahlii C-01, Clostridium Ijungdahlii O-52, Clostridium magnum, Clostridium pasteurianum, Clostridium ragsdalei P11, Clostridium scatologenes, Clostridium thermoaceticum, Clostridium ultunense, Desulfotomaculum kuznetsovii, Eubacterium limosum, Geobacter sulfurreducens, Methanosarcina acetivorans, Methanosarcina barkeri, Moorella thermoacetica, Moorella thermoautotrophica, Oxobacter pfennigii, Peptostreptococcus productus, Ruminococcus productus, Thermoanaerobacter kivui, and mixtures thereof, as described in U.S. Pat. Nos. 8,592,191 and 11,186,811 to Bell, et al. The method of Bell, et al., uses syngas in which the CO/CO₂ molar ratio may be at least 0.75, and the syngas is at least 20 mole % or at least 50 mole % CO, or at least 60 mole % CO, or at least 70 mole % CO. Strains of Alkalibaculum bacchi are also used in Liu, et al., "Fermentative production of ethanol from syngas using novel moderately alkaliphilic strains of Alkalibaculum bacchi," Bioresource Technology, Vol. 104, pp. 336-341 (January 2012). Other fermentation methods are described in Michael Köpke, et al., "Fermentative production of ethanol from carbon monoxide," Current Opinion in Biotechnology, Vol. 22, Issue 3, pp. 320-325 (June 2011). See, also, US 2010/0227377 A1; US 2010/0317074 A1; US 2015/0337343 A1; and US 5,173,429.

Alternatively, to form ethanol, the hydrogen and carbon monoxide of the synthesis gas are first reacted to form methanol, which is dehydrated to produce an ether, such as dimethyl ether. The ether(s) formed is/are subjected to carbonylation with unreacted carbon monoxide from the synthesis gas to provide an acetate, such as methyl acetate. The acetate(s) formed is/are subjected to hydrogenolysis to produce ethanol. See, e.g., US 9,115,046 B2.

Conversion of ethanol and methanol to aromatics can be performed using zeolite based catalysts, such as those based on ZSM-5 and/or ZSM-11. See, for example, Wang, et al., "Conversion of Methanol to Aromatics in Fluidized Bed Reactor," Catalysis Today, 233, 8-13 (2014); Juybar, et al., "Conversion of Methanol to Aromatics Over ZSM-5/11 Intergrowth Zeolites and Bimetallic Zn-Cu-ZSM-5/11 and Ga-Ag-ZSM-5/11 Catalysts Prepared with Direct Synthesis Method," J. Chem. Sci. 131:104 (2019); Xu, et al., "Effect of Metal on the Methanol to Aromatics Conversion Over Modified ZSM-5 in the Presence of Carbon Dioxide," RSC Adv. 7, 10729-10736 (2017); Gong, et al., "High-Efficiency Conversion of Methanol to BTX Aromatics Over a Zn-Modified Nanosheet-HZSM-5 Zeolite," Ind. Eng. Chem. Res. 60, 1633-1641 (2021); Shoinkhorova, et al., "Highly Selective and Stable Production of Aromatics via High-Pressure Methanol Conversion," ACS Catal. 11, 3602-3613 (2021); Hamieh, et al., "Methanol and Ethanol Conversion into Hydrocarbon over H-ZSM-5 Catalyst," Eur. Phys. J. Special Topics, 224, 1817-1830 (2015); Inaba, et al., "Ethanol Conversion to Aromatic Hydrocarbons over Several Zeolite Catalysts," React. Kinet. Catal. Lett., 88, 135-142 (2006); Ivanovna, et al., "Effect of Temperature on Ethanol Conversion over the Surface of Zr-modified Zeolite ZSM-5," Catal. Sustain. Energy, 2, 83-86 (2015).

See, also, A. M. Niziolek, et al., "Biomass-Based Production of Benzene, Toluene, and Xylenes via Methanol: Process Synthesis and Deterministic Global Optimization," Energy & Fuels, 30, 4970-4998 (2016); and Narula, et al., "Heterobimetallic Zeolite, InV-ZSM-5, Enables Efficient Conversion of Biomass Derived Ethanol to Renewable Hydrocarbons," Scientific Reports 5:16039 (2015).

### Synthesis of Aniline from Benzene (S114)

The method for synthesizing aniline from benzene is not particularly limited and conventionally known methods may be employed for conversion of the benzene produced as described above. In one embodiment, a two-step method may be employed. In the first step, benzene (and/or a mono, di, or tri-C₁-C₆ alkyl-substituted benzene) is converted to nitrobenzene (and/or a mono, di, or tri-C₁-C₆ alkyl-substituted nitrobenzene). For example, benzene is reacted with an acid mixture of concentrated nitric acid and concentrated sulfuric acid to form nitrobenzene:

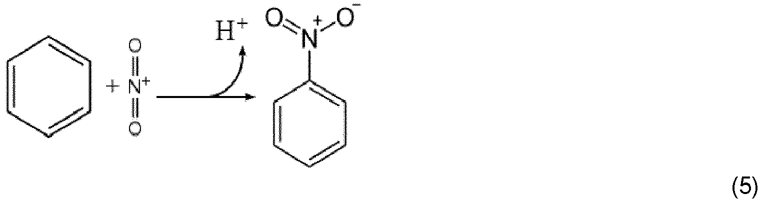

In the second step, the resulting nitrobenzene is reduced to aniline by a reduction technique such as Béchamp reduction or catalytic reduction:

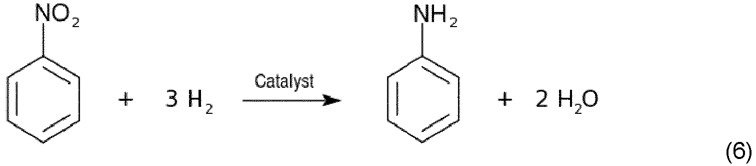

For example, the reduction may include stirring nitrobenzene with hydrochloric acid and powdered iron (as the catalyst) at a suitable temperature, such as 100°C.

Catalytic reduction of nitrobenzene to aniline can be performed with a mixture of CO/H₂O as a reducing agent in the presence of a palladium (II) catalyst. See, Krogul-Sobczak, et al., "Reduction of Nitrobenzene to Aniline by CO/H2O in the Presence of Palladium Nanoparticles," Catalysts, 9(5), 404 (2019).

Nitrobenzene can also be selectively reduced to aniline by aqueous methyl formate in the presence of a catalytic system which includes [Ru₃(CO)₁₂], Pd(OAc)₂, tricyclohexylphosphine, and 1,10-phenanthroline. See, e.g., Ben Taleb, et al., "Catalytic reduction of nitrobenzene to aniline with aqueous methyl formate," J. Organometallic Chem., 456(2) 263-2697 (1993).

In another method, aniline is prepared from benzene in a single direct one-step amination using ammonium hydroxide as an ammoniation agent, hydrogen peroxide is as oxygenant, in the presence of a titanium silicalite-1 (TS-1)-based catalyst, such as TS-1 with Ni, Cu, Ce, or V, at a temperature of 40-80 °C in a suitable solvent, such as acetonitrile, trimethyl carbinol, or *N*,*N*-dimethylacetamide. See, e.g., CN 101906045 A.

### Synthesis of Rubber-Forming Additives from Aniline (S116)

Example uses of the syngas-derived aniline (and/or a mono, di, or tri-C₁-C₆ alkyl-substituted aniline) include the synthesis of vulcanizing accelerators for the vulcanization of rubber and synthesis of anti-degradants/antioxidants.

### 1. Synthesis of Vulcanization (Cure) Accelerators from Aniline

A vulcanization accelerator is a chemical added into a rubber compound to increase the speed of vulcanization (curing) and/or to permit vulcanization to proceed at lower temperature and with greater efficiency. Vulcanization accelerators can also decrease the amount of sulfur needed for vulcanization.

In one embodiment, the vulcanization accelerator is one which includes a thiazole group (or groups). Example vulcanization accelerators with a thiazole group include 2-mercaptobenzothiazole (MBT) and dibenzothiazyl disulfide. MBT, for example, can be synthesized through the reaction of aniline with carbon disulfide in the presence of sulfur.

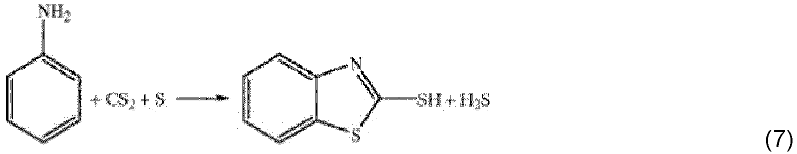

The process can be carried out as described in US 1,631,871 or US 5,367,082A for example. Oxidation of the 2-mercaptobenzothiazole formed gives dibenzothiazyl disulfide, as described, for example in US 4,463,178 A.

MBT can be used as an intermediate in the formation of other thiazole group-containing cure accelerators, such as (2-2'-dithiobis(benzothiazole)) and (2-(4-morpholinothio)-benzothiazole); and sulfanemide-group containing accelerators, such as (*N*,*N*'-dicyclohexyl-2-benzothiazylsulfenamide), (*N*-cyclohexyl-2-benzothiazylsulfenamide), (*N*-*tert*-butyl-2-benzothiazylsulfenamide), and (2-benzothiazylsulfenamide). For example, US 4,755,608 A and US 4,859,778 A describe processes for preparing (2-2'-dithiobis(benzothiazole)) from MBT. Processes for forming sulfanemides from MBT are described, for example, in US 3,658,808 A.

Other examples of cure accelerators are diaryl guanidines, such as diphenyl guanidine, which can be prepared by reaction of cyanogen chloride with an aqueous solution of aniline (and/or a respective alkyl-substituted aniline) followed by neutralization with sodium hydroxide. This process is described, for example, in US 4,898,978 and US 4,739,117.

Guanidine-based cure accelerators are often used as secondary accelerators to activate primary accelerators, such as thiazole- and sulfenamide-group containing accelerators. The dosages of the secondary accelerators are generally between 10-40% of the primary accelerator.

An example vulcanization system including one or more of the exemplary syngas-derived vulcanization accelerators may include:

| | | | |
|---|---|---|---|
| Zinc oxide | 3.0 | to | 10 phr |
| Stearic acid | 0.5 | to | 4 phr |
| Accelerator(s) | 0.3 | to | 6 phr |
| Sulfur | 0.5 | to | 4 phr |

where the parts per hundred (phr) total rubber are exemplary.

In an exemplary embodiment, at least 20 wt. % or at least 50 wt. % or at least 80 wt. % or up to 100 wt. % of the cure accelerator(s) used in the vulcanization system, is/are derived from syngas, in particular, via the conversion of syngas to aniline (and/or an alkyl-substituted aniline) via benzene (and/or an alkyl-substituted benzene), as exemplified by the methods described above.

### 2. Synthesis of Anti-degradants from Aniline

The anti-degradant can be selected from antiozonants, antioxidants, and compounds having both of these functions.

Aniline can be used to form 4-aminodiphenylamine (4-ADPA), which is a dimer of aniline that can be used to form various anti-degradants. Methods for preparing 4-ADPA include:
(a) reaction of p-nitro-chlorobenzene with aniline in the presence of a catalyst to produce 4-nitrodiphenylamine, then reduction of 4-nitrodiphenylamine to 4-aminodiphenylamine with sodium sulfide (see, for example, US 6,815,562);
(b) reaction of formic acid with aniline to prepare formanilide, which in turn reacts with p-nitro-chlorobenzene in the presence of an acid-binding agent, such as potassium carbonate, to produce 4-nitrodiphenylamine. 4-nitrodiphenylamine is reduced by sodium sulfide to form 4-aminodiphenylamine (see, for example, US 4,122,118; US 4,140,716; US 4,155,936; US 4,187,248; US 4,187,249; US 4,196,146; US 4,209,463; US 4,670,595; US 4,614,817; US 4,683,332; and US 4,782,185);
(c) nitrosation of diphenylamine using a nitrite in an organic solvent to produce N-nitrosodiphenylamine, which is rearranged to 4-nitrosodiphenylamine hydrochloride under the action of anhydrous hydrogen chloride. 4-nitrosodiphenylamine hydrochloride is neutralized with a base to give 4-nitrosodiphenylamine, which is reduced to 4-aminodiphenylamine by sodium sulfide. (See, e.g., US 4,518,803);
(d) reacting nitrobenzene and aniline in presence of a complex base catalyst to form a reaction mixture, and hydrogenating the reaction mixture to obtain 4-aminodiphenylamine. An example complex base catalyst includes a tetraalkyl ammonium hydroxide, a tetraalkyl ammonium salt, and an alkali metal hydroxide or oxide, as described, for example, in US 8,293,673 B2; and
(e) direct coupling of aniline and nitrobenzene in the presence of a base to give a mixture of 4-nitrosodiphenylamine and 4-nitrodiphenylamine, accompanied by some azobenzene and phenazine as by-products. The coupling reaction is followed by catalytic hydrogenation in presence of water to give 4-ADPA and regenerated base. The base is separated as part of an aqueous layer which is recycled to the process. 4-ADPA is obtained by fractional distillation of the organic phase (see, e.g., US 5,117,063; US 5,453,541; US 5,608,111; US 5,623,088; US 5,739,403; US 5,977,411; US 6,140,538; US 6,365,933; US 6,495,723; US 6,583,320; US 6,770,189; US 7,084,302; US 7,176,333; US 7,183,439; and US 7,235,694).

Other example methods for preparation of 4-aminodiphenylamine from aniline are described in Králik, et al., "The Story of 4-Aminodiphenylamine," Proc. 6th Int'l Conf. on Chemical Technology, pp. 319-325 (2018).

4-aminodiphenylamine can be reacted with acetone or methyl isobutyl ketone in the synthesis of *N*-(1,3-dimethylbutyl)-*N*'-phenyl-1,4-benzenediamine (6PPD). See, e.g., US 2015/0045584 A1. In one method, diacetone alcohol, which can be synthesized by, for example, aldol condensation of two molecules of acetone, is dehydrated to be converted to mesityl oxide. Then, hydrogenation of the mesityl oxide with a palladium catalyst or the like gives methyl isobutyl ketone, which is reacted with 4-aminodiphenylamine as described above. 6PPD can be used as an antioxidant and/or antiozonant in rubber compounds.

Aniline can also be used as a starting material for the synthesis of other suitable anti-degradants, such as trimethyl-dihydroquinolines (TMQs). TMQs are anti-degradants based on polymerized aniline-acetone condensation products. See, for example, US 4,897,482. The individual products differ by the degree of polymerization. Benzenediamines and toluenediamines, such as 1,4-benzene diamine (also called *p-*phenylenediamine) can be prepared by reacting aniline with ammonia. See, for example, Robert A. Smiley, "Phenylene- and Toluendiamines" in Ullmann's Encyclopedia of Industrial Chemistry, pp. 617-622 (15 June 2000). In the Du Pont process, for example, aniline is diazotized using nitrogen oxides. The diazo compound reacts with excess aniline to produce 1,3-diphenyltriazine, which rearranges under acidic conditions to 4-aminoazobenzene. 4-aminoazobenzene is catalytically cleaved and hydrogenated to give p-phenylenediamine and aniline. The aniline can be recovered and recycled.

Other example antioxidants which can be formed from aniline include quinoline antioxidants, such as 2,2,4-trimethyl-1,2-dihydroquinoline polymers. 2,2,4-trimethyl-1,2-dihydroquinoline polymers can be synthesized from aniline by feeding acetone, as needed, at a temperature of 80 to 150°C, e.g., 140°C, in the presence of an acidic catalyst. See, e.g., US 4,746,743 A.

In an exemplary embodiment, at least 20 wt. % or at least 50 wt. % or at least 80 wt. % or up to 100 wt. % of the antioxidant(s) used forming the tire, is/are derived from syngas, in particular, via the conversion of syngas to aniline (and/or an alkyl-substituted aniline) via benzene (and/or an alkyl-substituted benzene), as exemplified by the methods described above.

Acetone used in the reactions above can be produced as follows:
1. Conversion of syngas (in particular, the CO component) to ethanol; and
2. Conversion of ethanol to acetone.

The first step may be performed by gas fermentation, as described in further detail below for S118. The second step may include one of the following processes:
(a) Heating the ethanol produced (e.g., in hydrated form) at a temperature of 400° C, or higher, in the presence of a Zr-Fe catalyst. The Zr-Fe catalyst may be an iron oxide (hematite)-based catalyst carrying Zr, in which the Zr content may be from 2 to 10 wt. %. See, for example, US 7,973,199 B2.
(b) Subjecting the ethanol to a conversion reaction by a mixed oxide catalyst, such as a ZnO/CaO catalyst, or the like. See, for example, US 2017/0226028 A1.
(c) using a dehydration reaction to synthesize ethylene from ethanol, then synthesizing propylene from the ethylene, and subjecting the propylene to a hydration reaction to prepare isopropanol, followed by dehydrogenation. See, e.g., US 4,605,776.

Acetone used in the synthesis of the antioxidants can also be synthesized, for example, as follows: a biomass material is subjected to acetone-butanol fermentation by a microorganism to give a mixed solvent of butanol, acetone and the like, and the mixed solvent is distilled so that acetone is obtained. Examples of the biomass material include cellulose, agricultural products and waste thereof, and sugars, and sugars are particularly preferred. The microorganism for the acetone-butanol fermentation is not particularly limited. Preferred examples of the microorganism include wild type, variants, or recombinants of a member selected from the group consisting of those belonging to the genus Escherichia, the genus Zymomonas, the genus Candida, the genus Saccharomyces, the genus Pichia, the genus Streptomyces, the genus Bacillus, the genus Lactobacillus, the genus Coryne, or the genus Clostridium. Microorganisms belonging to the genus Clostridium are particularly suitable, such as Clostridium acetobutylicum, Clostridium beijerinckii, Clostridium saccharobutylicum, and Clostridium saccharoperbutylacetonicum.

Moreover, microorganisms containing a gene encoding acetoacetate decarboxylase (EC4.1.1.4), coenzyme A transferase, or thiolase from a microorganism of the genus Clostridium may be used.

Acetone may also be obtained by dry distillation of wood to give pyroligneous acid, followed by further fractional distillation or separation by liquid chromatography or the like.

Furthermore, acetone can be synthesized by pyrolyzing cellulose in a wood material to give acetic acid, neutralizing the acetic acid with calcium hydroxide to give calcium acetate, and then pyrolyzing the calcium acetate. Since acetic acid is generated by oxidation of ethanol during the fermentation in the synthesis of bioethanol, the acetic acid can be utilized to synthesize acetone through the same process as above.

### Synthesis of Naphtha or Ethanol from Syngas (S118)

In one embodiment, naphtha is generated from syngas by chemical conversion, as described above in S112.

In one embodiment, ethanol is generated from syngas by fermentation or chemical conversion, as described above in S112.

### Synthesis of Synthetic Rubber from Ethanol (S120-S122)

Examples of suitable synthetic rubbers for a tire which can be synthesized from ethanol (e.g., as produced at S118) include styrene butadiene rubber (SBR), butadiene rubber (BR) and emulsion butadiene rubber (EBR).

Styrene butadiene rubber can be made entirely from syngas. Butadiene can be derived from syngas via ethanol or direct gas fermentation while styrene can be from syngas via benzene. In one embodiment, 1,3-butadiene is produced at S120 by reacting ethanol, generated from syngas, at a high temperature in the presence of a solid acid catalyst, such as zeolites, alumina, titanium compounds, sulfate ion-supported zirconia, and WO₃-supported zirconia. In another embodiment, 1,3-butadiene is obtained by oxidizing ethanol to form acetaldehyde, followed by addition of additional ethanol in the presence of a tantalum/silicon dioxide catalyst and then heating. This allows synthetic rubbers for a tire to be produced from waste materials. In another embodiment, the ethanol used in this process is bioethanol, which is derived from biomass (plant materials). Both these approaches reduce or eliminate the need for using petroleum-based materials to form synthetic rubbers.

SBR can be produced at S122 by copolymerization of styrene and 1,3-butadiene. BR can be produced by polymerization of 1,3-butadiene.

The styrene used for forming the styrene-butadiene rubber can be formed by ethylation of benzene by Friedel-Crafts reaction or the like, followed by dehydrogenation of the resulting ethylbenzene with an iron catalyst or the like. The ethylene used in Friedel-Crafts reaction can be produced, for example, by dehydration of ethanol, for example.

### Conversion of ethanol or naphtha to reinforcing materials (S124)

Reinforcing materials for tires can also be derived from biomass. Polyethylene terephthalate (PET), for example, can be formed from ethylene glycol and terephthalic acid. One or both of these can be derived from biomass. For example, ethylene glycol can be produced from bio-ethanol, e.g., produced as described above and terephthalic acid can be produced from bio-isobutanol. See, for example, Damayanti, et al., "Conversion of Lignocellulose for Bioethanol Production, Applied in Bio-Polyethylene Terephthalate," Polymers (Basel), 13(17): 2886 (2021). See, also, Satapathy, et al., "Synthesis of Ethylene Glycol from Syngas via Oxidative Double Carbonylation of Ethanol to Diethyl Oxalate and Its Subsequent Hydrogenation," ACS Omega, 3 (9) pp. 11097-11103 (2018), which describes a two-step process for formation of ethylene glycol. In the first step, diethyl oxalate is selectively synthesized via oxidative double carbonylation of ethanol and carbon monoxide (CO) using a ligand-free, recyclable Pd/C catalyst. In the second step, the diethyl oxalate produced undergoes subsequent hydrogenation using [2-(di-tert-butylphosphinomethyl)-6-(diethylaminomethyl)pyridine]ruthenium(II) chlorocarbonyl hydride to form ethylene glycol and ethanol. The generated ethanol can be recycled back to the first step for double carbonylation.

Para-xylene, a starting material for forming terephthalic acid, can also be generated in a one-pass conversion of syngas using a hybrid catalyst, Cr/Zn-Zn/Z5@S1. See, e.g., Zhang, et al., "One-pass selective conversion of syngas to para-xylene," Chem Sci., 8 (12) pp. 7941-7946 (2017). Zhang notes that para-xylene can also be produced by the catalytic reforming of naphtha.

In the Amoco process, for example, terephthalic acid is produced by catalytic oxidation of p-xylene:

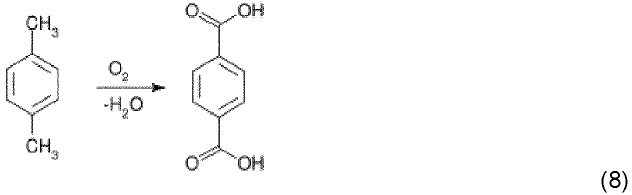

### Forming a Tire (S126)

The rubber-forming additives for a tire and the synthetic rubbers for a tire obtained as above may be used for preparing rubber compositions for a tire or portion(s) thereof (e.g., treads, sidewalls, etc.). The tire-forming composition may include one or more synthetic and/or natural rubbers. Examples of synthetic rubber for a tire include styrene butadiene rubber (SBR), and butadiene rubber (BR).

In addition to the above polymer component(s), the rubber compositions generally contain other compounding ingredients usually used in the rubber industry, such as inorganic fillers (e.g., carbon black, silica, clay, aluminum hydroxide, calcium carbonate), silane coupling agents, process oil, softeners, vulcanization accelerators, and vulcanization accelerating auxiliaries. The rubber compositions may partially contain antioxidants, vulcanization accelerators and synthetic rubbers, which are derived from biomass, as described above.

The rubber compositions may be produced by known methods. For example, the composition can be produced by kneading the components with a rubber kneader such as an open roll mill, a Banbury mixer, and an internal mixer, and vulcanizing the resulting mixture.

A pneumatic tire can be produced by a conventional method using the rubber composition. Specifically, an unvulcanized rubber composition containing the components as needed is extruded and processed into the shape of a tire component, and then subjected to molding on a tire building machine to form an unvulcanized tire.

Thereafter, the unvulcanized tire is subjected to heat and pressure in a vulcanizer to produce a tire.

Use of the aniline prepared as above contributes to reduction in the amount of petroleum resources used in the production of rubber chemicals for a tire, such as antioxidants and vulcanization accelerators, and furthermore it enables production of such rubber chemicals for a tire without using petroleum resources.

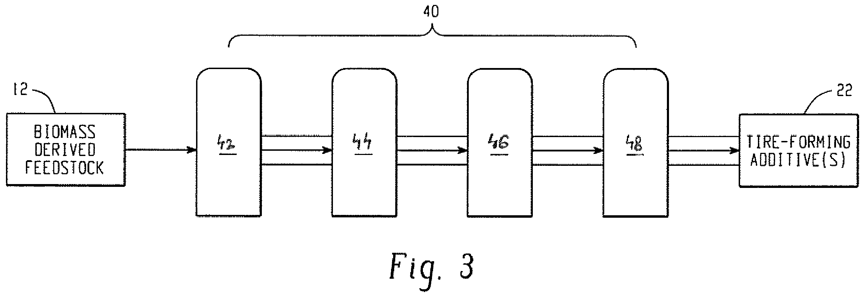

As illustrated in FIGURE 3, a system 40 for forming a tire forming additive includes a sequence of reactors which may include a first reactor (or reactors) 42 in which optionally-preprocessed biomass is converted to syngas, as described with reference to S106; a second reactor (or reactors) 44, fluidly connected to the first reactor(s) and configured for synthesizing at least one of benzene and an alkyl-substituted benzene from carbon monoxide and hydrogen in the syngas, as described with reference to S112; a third reactor (or reactors) 46, fluidly connected to the second reactor(s) and configured for synthesizing at least one of aniline and an alkyl-substituted aniline from the at least one of the benzene and the alkyl-substituted benzene, as described with reference to S114; a fourth reactor (or reactors) 48 fluidly connected to the third reactor and configured for synthesizing a tire-forming additive 22 from the at least one of the aniline and the alkyl-substituted aniline, the tire-forming additive being selected from an anti-degradant, a vulcanization accelerator, and combinations thereof. As will be appreciated, additional reactors may be incorporated into the sequence or feed into or out from reactor in the sequence. The additional reactors may be configured for performing one or more of steps S110, S118, S120, S122, and S124.

## Claims

1. A method of synthesizing an additive for a tire, the method comprising:
converting biomass to syngas or to a gaseous mixture predominately comprising carbon monoxide and hydrogen;
synthesizing at least one of benzene and an alkyl-substituted benzene from carbon monoxide and hydrogen in said syngas or said gaseous mixture;
synthesizing at least one of aniline and an alkyl-substituted aniline from the at least one of the benzene and the alkyl-substituted benzene; and
synthesizing the additive from the at least one of the aniline and the alkyl-substituted aniline.

2. The method of claim 1 wherein the additive is selected from the group consisting of an anti-degradant such as an antioxidant, antiozonant or mixtures thereof, a vulcanization accelerator, and combinations thereof.

3. The method of claim 2, wherein the synthesis of the anti-degradant comprises synthesizing 4-aminodiphenylamine from the at least one of the aniline and the alkyl-substituted aniline and synthesizing the anti-degradant from the 4-aminodiphenylamine.

4. The method of claim 2 or 3, wherein the anti-degradant is selected from the group consisting of N-(1,3-dimethylbutyl)-N'-phenyl-1,4-benzenediamine, 1,4-benzenediamine, N,N'-phenol derivatives of 4-aminodiphenylamine, tolyl derivatives of 4-aminodiphenylamine, trimethyl-dihydroquinolines, and mixtures thereof.

5. The method of claim 2, wherein (i) the vulcanization accelerator is selected from the group consisting of: thiazole-group containing compounds, sulfanemide-group containing compounds, diaryl guanidines, and mixtures thereof; or wherein (ii) the vulcanization accelerator is selected from the group consisting of: thiazole-group containing compounds selected from the group consisting of 2-mercaptobenzothiazole,dibenzothiazyl disulfide, (2-2'-dithiobis(benzothiazole)) and (2-(4-morpholinothio)-benzothiazole); sulfanemide-group containing compounds selected from the group consisting of (N,N'-dicyclohexyl-2-benzothiazylsulfenamide), (N-cyclohexyl-2-benzothiazylsulfenamide), (N-tert-butyl-2-benzothiazylsulfenamide), and (2-benzothiazylsulfenamide); diaryl guanidines; and mixtures thereof.

6. The method of at least one of the previous claims, wherein (i) the synthesis of the at least one of the benzene and the alkyl-substituted benzene comprises catalytic conversion of the carbon monoxide and hydrogen in said syngas or said gaseous mixture with a catalyst system comprising a zeolite-based catalyst; or wherein (ii) the synthesizing of the at least one of benzene and the alkyl-substituted benzene is performed with a zeolite-based catalyst.

7. The method of claim 6, wherein the catalyst system further comprises a zinc-based catalyst.

8. The method of at least one of the previous claims, wherein the synthesis of the at least one of the benzene and the alkyl-substituted benzene comprises conversion of the carbon monoxide and hydrogen in said syngas or said gaseous mixture to naphtha and reforming the naphtha to produce benzene.

9. The method of at least one of the previous claims, wherein the synthesizing of the at least one of aniline and the alkyl-substituted aniline comprises:
synthesizing at least one of nitrobenzene and an alkyl-substituted nitrobenzene from the at least one of the benzene and the alkyl-substituted benzene; and
synthesizing the at least one of aniline and the alkyl of the aniline from the at least one of the nitrobenzene and the alkyl-substituted nitrobenzene.

10. The method of at least one of the previous claims, further comprising (i) modifying an H₂/CO molar ratio in said syngas or said gaseous mixture; and/or further comprising (ii) reducing a sulfur content of said syngas or said gaseous mixture; and/or further comprising (iii) separating ash from said syngas or said gaseous mixture.

11. The method of at least one of the previous claims, wherein the biomass is selected from the group consisting of: hay, corn husks, wheat, switch grass, miscanthus, primary sludge from wood pulp, wood chips, seed oils, plant fruits, waste cooking oil, lychee fruit bark, compost, yard wastes, construction waste, demolition waste, treated sewage sludge, and mixtures thereof.

12. The method of at least one of the previous claims, wherein the method further comprises (i) synthesizing a rubber polymer from said syngas or said gaseous mixture and combining the rubber polymer with the additive; or wherein the method further comprises (ii) synthesizing a reinforcing material from said syngas or said gaseous mixture and combining the reinforcing material with a rubber composition derived from the additive and a rubber-forming polymer or with a rubber composition comprising the additive.

13. A method of forming a curing system comprising combining the additive in the form of a vulcanization accelerator as synthesized with the method in accordance with at least one of the previous claims with zinc oxide, stearic acid and sulfur.

14. A method of forming a tire or a tire component comprising combining the additive as synthesized with the method in accordance with at least one of the previous claims with a rubber-forming polymer.

15. A system for synthesizing an additive for a tire, the system (40) comprising:
a first reactor (42) in which biomass can be converted to syngas or to a gaseous mixture predominately comprising carbon monoxide and hydrogen;
a second reactor (44) fluidly connected to the first reactor and configured for synthesizing at least one of benzene and an alkyl-substituted benzene from carbon monoxide and hydrogen in said syngas or said gaseous mixture;
a third reactor (46) fluidly connected to the second reactor and configured for synthesizing at least one of aniline and the alkyl-substituted aniline from the at least one of the benzene and the alkyl-substituted benzene; and
a fourth reactor (48) fluidly connected to the third reactor and configured for synthesizing the additive from the at least one of the aniline and the alkyl-substituted aniline.
